(19) Europäisches Patentamt
European Patent Office
Office européen des brevets

(11) Veröffentlichungsnummer: **0 492 318 A2**

# (12) EUROPÄISCHE PATENTANMELDUNG

(21) Anmeldenummer: **91121422.9**

(22) Anmeldetag: **13.12.91**

(51) Int. Cl.5: **C07H 19/01**, A61K 31/71, C07D 311/74, C12N 1/14, C12P 17/06, C12P 19/44, //(C12P17/06,C12R1:77), (C12P19/44,C12R1:77)

(30) Priorität: **15.12.90 DE 4040147**

(43) Veröffentlichungstag der Anmeldung: **01.07.92 Patentblatt 92/27**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Zeeck, Axel, Prof. Dr.**
**Brüder-Grimm-Allee 22**
**W-3400 Göttingen(DE)**
Erfinder: **Breiding-Mack, Sabine, Dr.**
**Bärenkopfstrasse 9**
**W-3384 Liebenburg(DE)**
Erfinder: **Göhrt, Axel**
**Arndtstrasse 3**
**W-3400 Göttingen(DE)**
Erfinder: **Grabley, Susanne, Dr.**
**Hölderlinstrasse 7**
**W-6240 Königstein(DE)**
Erfinder: **Granzer, Ernold, Dr. Dr.**
**Falkensteiner Strasse 24**
**W-6233 Kelkheim (Taunus)(DE)**
Erfinder: **Hütter, Klaus, Dr.**
**Robert-Stolz-Strasse 3**
**W-6232 Bad Soden am Taunus(DE)**
Erfinder: **Thiericke, Ralf, Dr.**
**Auestrasse 35**
**W-6057 Dietzenbach(DE)**
Erfinder: **Wink, Joachim, Dr.**
**Magdeburger Strasse 14**
**W-6074 Rödermark(DE)**

(54) **Agistatine, neue Metabolite aus Fusarium sp., Verfahren zu ihrer Herstellung und ihrer Verwendung.**

(57) Mit Hilfe eines Fusarium-Stammes können neue Metabolite der folgenden Formel I hergestellt werden

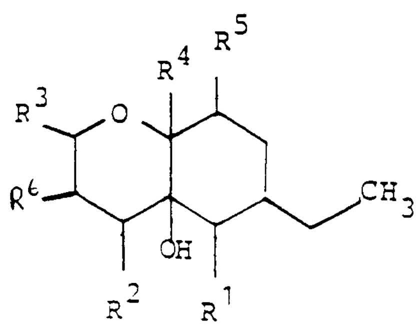

in der
$R^1$ Wasserstoff oder Hydroxyl ist

EP 0 492 318 A2

$R^2$ Hydroxyl oder eine Oxogruppe bedeutet
$R^3$ Methoxy oder Hydroxyl bedeutet oder zusammen mit $R^1$ eine Etherbrücke bildet und
$R^4$ Wasserstoff oder Hydroxyl ist
$R^5$ Wasserstoff bedeutet oder zusammen mit $R^4$ eine zusätzliche Bindung darstellt und
$R^6$ Wasserstoff oder zusammen mit $R^3$ eine zusätzliche Bindung ist.

Die Metabolite sind insbesondere zur Inhibierung der Cholesterinbiosynthese einzusetzen.

Es wurde gefunden, daß Fusarium-Stämmeneue Metabolite, die Agistatine produzieren können, die pharmakologische und damit therapeutische Wirksamkeit besitzen und insbesondere vorteilhaft als Inhibitor der Cholesterin-Biosynthese mit entsprechendem pharmakologischen Nutzen eingesetzt werden können.

Die Erfindung betrifft somit:

1. Verbindungen der Formel

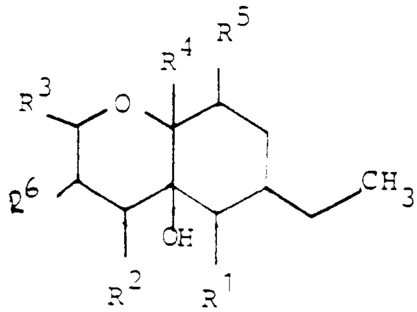

I

in der

R[1] Wasserstoff oder Hydroxyl ist
R[2] Hydroxyl oder eine Oxogruppe bedeutet
R[3] Methoxy oder Hydroxyl bedeutet oder zusammen mit R[1] eine Etherbrücke bildet und
R[4] Wasserstoff oder Hydroxyl ist
R[5] Wasserstoff bedeutet oder zusammen mit R[4] eine zusätzliche Bindung darstellt und
R[6] Wasserstoff oder zusammen mit R[3] eine zusätzliche Bindung ist.

2. Ein Verfahren zur Herstellung der Verbindungen der unter 1. charakterisierten Formel I, das dadurch gekennzeichnet ist, daß Fusarium DSM 5983 in einem Nährmedium kultiviert wird, bis sich die Verbindungen der Formel I in der Kultur anhäuft.

3. Eine Verwendung der Verbindungen der allgemeinen Formel I als Inhibitor der Cholesterin Biosynthese sowie deren pharmakologische Nutzung.

Im folgenden wird die Erfindung detailliert beschrieben, insbesondere in ihren bevorzugten Ausführungsformen. Ferner wird die Erfindung durch den Inhalt der Patentansprüche bestimmt.

Die erfindungsgemäßen Verbindungen werden bevorzugt mit Fusarium sp. DSM 5983 hergestellt. Der Stamm wurde aus einer Erdprobe aus Hawaii isoliert und bei der Deutschen Sammlung von Mikroorganismen nach den Regeln des Budapester Vertrages am 06.06.1990 unter obengenannter Nummer hinterlegt.

Der Stamm Fusarium sp. DSM 5983 besitzt weißes Luftmycel und weiße Konidien. Er bildet die für Fusarien charakteristischen Makro- und Mikrokonidien sowie Chlamydosporen.

In einer Nährlösung, die eine Kohlenstoffquelle und eine Stickstoffquelle sowie die üblichen anorganischen Salze enthält, produziert Fusarium sp., bevorzugt DSM 5983, die Verbindungen der allgemeinen Formel I. Anstelle des Stammes DSM 5983 können natürlich auch dessen Mutanten und Varianten eingesetzt werden, soweit sie diese Verbindung synthetisieren. Solche Mutanten können in an sich bekannter Weise durch physikalische Mittel, beispielsweise Bestrahlung, wie mit Ultraviolet- oder Röntgenstrahlen, oder chemische Mutagene, wie beispielsweise Ethylmethansulfonat (EMS), 2-Hydroxy-4-methoxybenzophenon (MOB) oder N-Methyl-N'-nitro-N-nitrosoguanidin (MNNG) erzeugt werden.

Als bevorzugte Kohlenstoffquellen für die aerobe Fermentation eignen sich assimilierbare Kohlenhydrate und Zuckeralkohole, wie Glukose, Laktoseoder D-Mannit sowie kohlenhydrathaltige Naturprodukte, wie Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung der Verbindungen der Formel I verläuft besonders gut in einer Nährlösung, die etwa 0,2 bis 5 %, bevorzugt 1 bis 4 %, Malzextrakt und 0,02 bis 0,5%, bevorzugt 0,1 bis 0,4 %, Hefeextrakt enthält, sowie 0,2 bis 5,0 %, bevorzugt 0,5 bis 2 % Glucose und 0,01 bis 0,1 %, bevorzugt 0,04 bis 0,06 % $(NH_4)_2HPO_4$, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 15 bis 30 °C,

vorzugsweise bei etwa 20 bis 30 ° C, insbesondere bei 23 bis 28 ° C durchgeführt werden. Der pH-Bereich sollte zwischen 1 und 7 liegen, vorteilhaft zwischen 2,5 und 4,5. Man kultiviert den Mikroorganismus unter diesen Bedingungen im allgemeinen über einen Zeitraum von 24 bis 300 Stunden, bevorzugt 36 bis 140 Stunden.

Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z. B., indem man ein versportes Mycel in eine Nährlösung überimpft und etwa 36 bis 120 Stunden, bevorzugt 48 bis 72 Stunden, wachsen läßt. Das versporte Mycel kann beispielsweise erhalten werden, indem man den Stamm etwa 3 bis 40 Tage, bevorzugt 4 bis 10 Tage, auf einem festen oder flüssigen Nährboden, beispielsweise Hefe-Malz-Agar oder Kartoffel-Dextrose-Agar, wachsen läßt.

Der Fermentationsverlauf kann anhand des pH-Wertes der Kultur oder des Mycelvolumens sowie durch chromatographische Methoden, wie z. B. Dünnschichtchromatographie oder High Pressure Liquid Chromatography, oder Ausprüfen der biologischen Aktivität überwacht werden. Die Verbindungen der allgemeinen Formel I sind sowohl im Mycel als auch im Kulturfiltrat enthalten.

Die Isolierung der genannten Verbindungen aus dem Kulturmedium erfolgt nach bekannten Methoden unter Berücksichtigung der chemischen, physikalischen und biologischen Eigenschaften der Produkte. Zum Testen der Metabolit-Konzentration im Kulturmedium oder in den einzelnen Isolierungsstufen kann die Dünnschichtchromatographie, beispielsweise an Kieselgel mit Butanol/Eisessig/Wasser oder Ethylacetat/Methanol/Wasser Mischungen als Laufmittel verwendet werden. Die Detektion bei der dünnschichtchromatographischen Auftrennung kann beispielsweise durch Färbereagentien wie Anisaldehyd oder durch biologische Testung, z. B. als Inhibitor der Cholesterin-Biosynthese erfolgen, wobei die Menge der gebildeten Substanz zweckmäßig mit einer Eichlösung verglichen wird.

Zur Isolierung der Verbindungen I werden Kulturbrühe und Mycel zuerst mit unpolaren organischen Lösungsmitteln, wie z. B. n-Hexan, Petrolether oder halogenierten Kohlenwasserstoffen wie z. B. Chloroform usw. extrahiert, um die unpolaren Verunreinigungen zu entfernen. Anschließend wird mit einem polareren organischen Lösungsmittel, beispielsweise niederen Alkoholen, Aceton und/oder Essigester sowie Gemischen dieser Lösungsmittel, extrahiert.

Die Reinisolierung der Verbindungen der Formel I erfolgt an geeigneten Materialien, vorzugsweise z. B. an Kieselgel, Aluminiumoxid, Ionenaustauschern oder Adsorberharzen, durch anschließende Elution mit organischen, polaren Lösungsmitteln oder Lösungsmittelgemischen, wie z. B. Essigsäurealkylester, Gemische aus Essigsäurealkylester mit einem niederen Alkanol, Chloroform oder Methylenchlorid bzw. Gemischen dieser Lösungsmittel mit niederen Alkanolen, gegebenenfalls auch mit Wasser, bzw. einem für Ionenaustauscher-Harzen geeigneten pH- bzw. Salzgradienten, wie beispielsweise Kochsalz oder Tris-(hydroxymethyl)-aminomethan-HCl(Tris-Puffer), und Vereinigung der biologisch aktiven Fraktionen.

Die Verbindungen der Formel I sind im festen Zustand und in Lösungen im pH-Bereich zwischen 1 und 9, insbesondere 3 und 7, stabil und lassen sich damit in übliche galenische Zubereitungen einarbeiten.

Die erfindungsgemäßen Verbindungen kann man als Lipidregulatoren, insbesondere zur Hemmung der Cholesterinbiosynthese, verwenden.

Die Verbindungen der Formel I können zur Prophylaxe und Behandlung von Krankheiten verwendet werden, die auf einem erhöhten Cholesterinspiegel beruhen, insbesondere koronare Herzkrankheiten, Arteriosklerose und ähnlicher Krankheiten. Die Anwendung betrifft auch pharmazeutische Zubereitungen der Verbindungen der Formel I.

Bei der Herstellung von Arzneimitteln können neben dem Wirkstoff auch pharmazeutisch unbedenkliche Zusatzstoffe, wie Verdünnungsmittel und/oder Trägermaterialien, verwendet werden. Hierunter sind physiologisch unbedenkliche Substanzen zu verstehen, die nach Vermischen mit dem Wirkstoff diesen in eine für die Verabreichung geeignete Form bringen.

Geeignete feste oder flüssige galenische Zubereitungsformen sind beispielsweise Tabletten, Dragees, Pulver, Kapseln, Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z. B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können, genannt.

Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelzubereitungen Verwendung finden. Es können auch pharmakologisch akzeptable polymere Träger wie z.B. Polyphenylpyrolidone, oder andere pharmazeutisch akzeptable Additive, wie z.B. Cyclodextrin oder Polysaccharide,

verwendet werden. Die Verbindungen können insbesondere auch mit Additiven, die Gallensäure binden, im besonderen nicht toxischen, im Gastrointestinal-Trakt nicht resorbierbaren, basischen Anionen-Austauschern, kombiniert werden.

Die Präparate können oral, rektal oder parenteral verabreicht werden. Vorzugsweise können die Präparate in Dosierungseinheiten hergestellt werden; insbesondere Tabletten, Kapseln, Suppositorien, stellen Beispiele für geeignete Dosierungseinheiten dar. Jede Dosierungseinheit, insbesondere für die orale Applikation, kann bis zu 1000 mg, bevorzugt jedoch 10 bis 100 mg, des aktiven Bestandteiles enthalten. Jedoch können auch darüber- oder darunterliegende Dosierungseinheiten verwendet werden, die gegebenenfalls vor Verabreichung zu teilen bzw. zu vervielfachen sind.

Gegebenenfalls können die Dosierungseinheiten für die orale Verabreichung mikroverkapselt werden, um die Abgabe zu verzögern, oder über einen längeren Zeitraum auszudehnen, wie beispielsweise durch Überziehen oder Einbetten des Wirkstoffs in Teilchenform in geeignete Polymere, Wachse oder dergleichen.

Die parenterale Verabreichung kann unter Verwendung flüssiger Dosierungsformen, wie steriler Lösungen und Suspensionen, erfolgen, die für die intramuskuläre oder subcutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension gegebenenfalls unter Mitverwendung von geeigneten Stabilisatoren, Emulgatoren und/oder Konservierungsmitteln und/oder Antioxidantien hergestellt.

Die orale Applikationsform ist, insbesondere unter dem Gesichtspunkt einer langen Therapiedauer, bevorzugt und stellt eine wesentliche Erleichterung in der Prävention und Therapie oben angeführter Krankheiten dar.

Die pharmazeutischen Präparate werden nach allgemein üblichen Verfahren hergestellt. Das Dosierungsschema kann vom Typ, Alter, Gewicht, Geschlecht und medizinischen Zustand des Patienten oder Risikogefährdeten abhängen.

In den folgenden Beispielen wird die Erfindung in weiteren Details beschrieben. Prozentangaben beziehen sich auf das Gewicht, wenn nicht anders angegeben.

Beispiele:

1.

a) Herstellung einer Sporensuspension des Produzentenstammes:

100 ml Nährlösung (60 g Malzextrakt, 2 g Hefeextrakt, 10 g Glucose, 0,5 g $(NH_4)_2HPO_4$ in 1 l Leitungswasser, pH-Wert vor der Sterilisation 6,0) in einem 500 ml sterilen Erlenmeyerkolben werden mit dem Stamm DSM 5983 beimpft und 72 Stunden bei 25 °C und 140 UpM auf einer rotierenden Schüttelmaschine inkubiert. Anschließend werden 20 ml Kulturflüssigkeit in einem sterilen 500 ml Erlenmeyerkolben mit dem Nährboden (Kartoffelinfus 4,0 g/l [Infus aus 200 g Kartoffeln], 20,0 g/l D-Glucose, pH vor der Sterilisation 5,6), dem zusätzlich 15 g Agar/l zur Verfestigung zugegeben wurde, gleichmäßig verteilt und dekantiert. Die Kulturen werden 10 bis 14 Tage bei 25 °C inkubiert. Die nach dieser Zeit entstandenen Sporen eines Kolbens werden mit 500 ml entionisiertem Wasser, das einen Tropfen eines handelsüblichen nichtionischen Tensids (z. B. ®Triton X 100, Fa. Serva) enthält, abgeschwemmt, sofort weiterverwendet oder bei -22 °C in 50 % Glycerin aufbewahrt.

b) Herstellung einer Kultur bzw. einer Vorkultur des Produzentenstammes im Erlenmeyerkolben:

Ein steriler 500 ml Erlenmeyerkolben mit 100 ml der unter a) beschriebenen Nährlösung wird mit einer auf einem Schrägröhrchen gewachsenen Kultur oder mit 0,2 ml Sporensuspension angeimpft und auf einer Schüttelmaschine bei 140 UpM und 25 °C inkubiert. Die maximale Produktion der Verbindungen der Formel I ist nach ca. 120 Stunden erreicht. Zum Animpfen von 10 und 100 l Fermentern genügt eine 72 Stunden alte Submerskultur (Animpfmenge ca. 5 %) aus der gleichen Nährlösung.

2. Herstellung der Verbindungen der allgemeinen Formel I:

Ein 10 l Fermenter wird unter folgenden Bedingungen betrieben:

| | |
|---|---|
| Nährmedium: | 20 g/l Malzextrakt<br>10 g/l Glucose<br>2 g/l Hefeextrakt<br>0,5 g/l $(NH_4)_2HPO_4$<br>pH 6,0 (vor der Sterilisation) |
| Inkubationszeit:<br>Inkubationstemperatur:<br>Rührergeschwindigkeit:<br>Belüftung: | 120 Stunden<br>25 °C<br>200 UpM<br>2 l Luft/min. |

Durch wiederholte Zugabe weniger Tropfen ethanolischer Polyollösung kann die Schaumbildung unterdrückt werden. Das Produktionsmaximum wird nach ca. 96 Stunden erreicht. Der pH-Wert beträgt bei der Ernte ca. 3,5. Die Ausbeuten liegen bei ca. 80 mg/l für die Verbindungen der Formel I.

3. Isolierung der Verbindungen der allgemeinen Formel I:

Nach Beendigung der Fermentation von DSM 5983 wird die Kulturbrühe unter Zusatz von ca. 2 % Filterhilfsmittel (z. B. Celite) filtriert. Es kann nach folgenden Schemata aufgearbeitet werden:

**Aufarbeitung/Isolierung**

Schema 1: Kulturüberstand

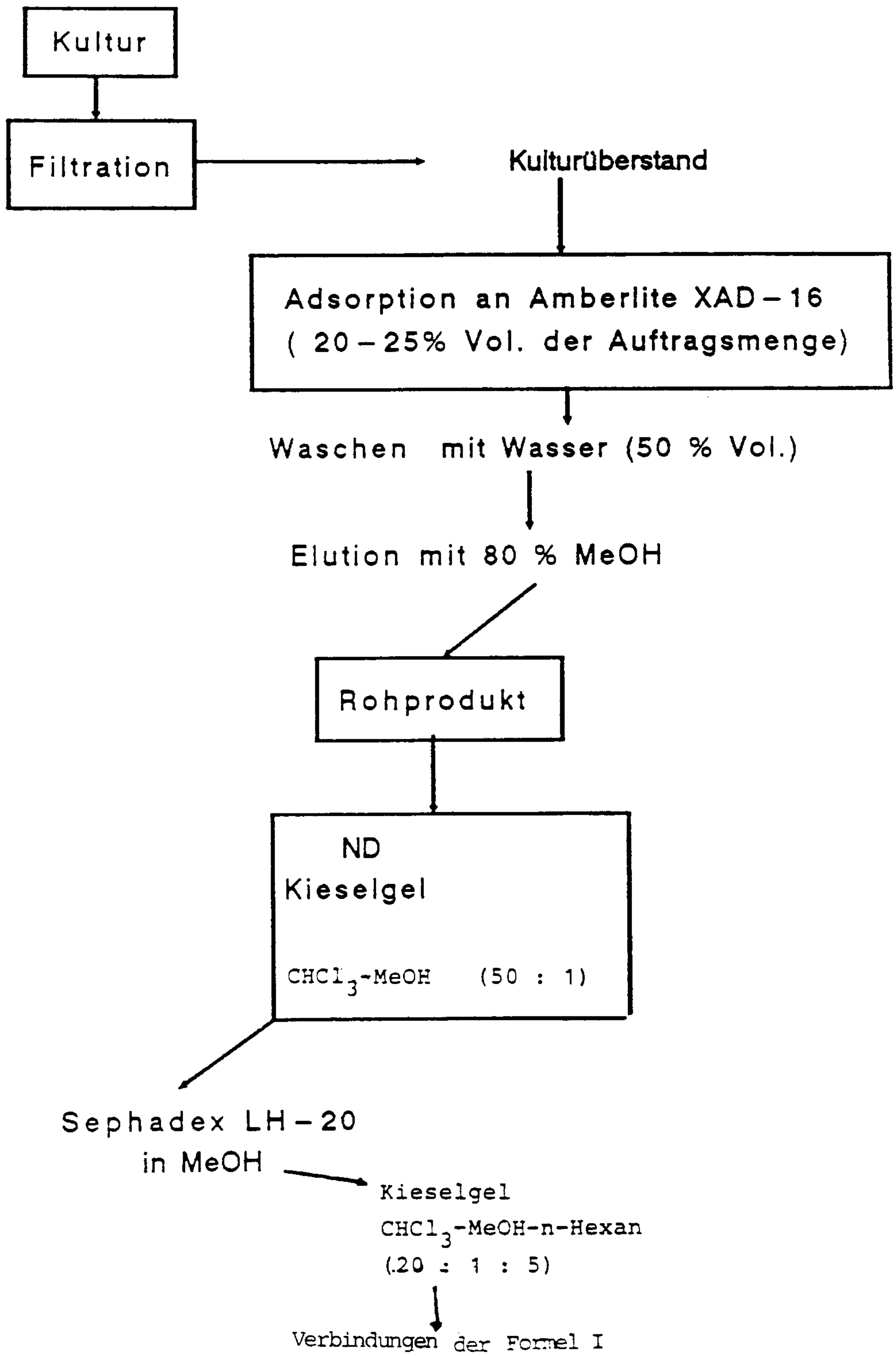

Aufarbeitung/Isolierung

Schema 1: Mycel

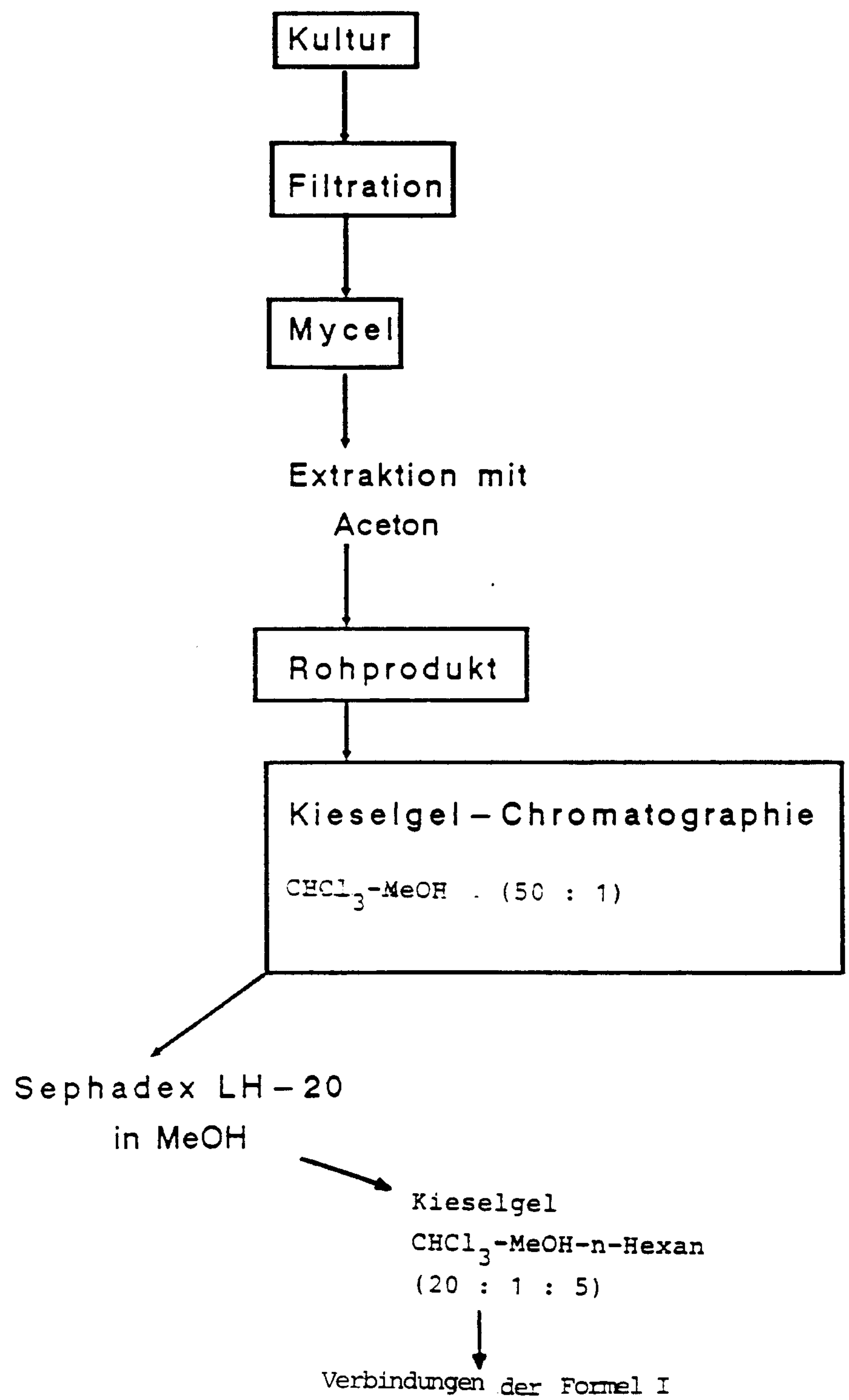


4. Biologische Testung als Cholesterinbiosynthese-Inhibitoren:

a) In Vitro-Bestimmung:

Monolayer von HEP-G2-Zellen in lipoproteinfreiem Nährmedium werden mit entsprechenden Konzentrationen der zu prüfenden Substanzen der allgemeinen Formel I eine Stunde vorinkubiert. Nach Zugabe der $^{14}C$-markierten Biosynthesevorstufe [$^{14}C$]Natriumacetat wird die Inkubation für 3 Stunden

fortgesetzt. Danach wird ein Teil der Zellen alkalisch verseift, bei vorheriger Zugabe eines internen Standards von $^{3}$H-Cholesterin. Die Lipide der verseiften Zellen werden mit einem Gemisch aus Chloroform-Methanol extrahiert. Dieses Lipidgemisch wird nach Zusatz von Trägercholesterin präparativ dünnschichtchromatographisch aufgetrennt, die Cholesterinbande nach Anfärbung isoliert und die aus dem $^{14}$C-Precursor gebildete Menge $^{14}$C-Cholesterin szintigraphisch bestimmt. In einem aliquoten Teil der Zellen wurde Zellprotein bestimmt, sodaß die in der Zelleinheit pro mg Zellprotein aus $^{14}$C-Vorläufer gebildete Menge $^{14}$C-Cholesterins berechnet werden kann. Zum Vergleich für die Hemmwirkung eines zugesetzten Prüfpräparates dient die Kontrolle, so daß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (Lichtmikroskopie) beurteilt und biochemisch durch Bestimmung der Laktat-Dehydrogenase Ausschüttung in das Inkubationsmedium gemessen. Als Standartpräparat wurde Lovastatin benutzt. Die Verbindungen der Formel I mit R = H hemmt die Cholesterin-Biosynthese in einer Konzentration von

$10^{-8}$ mol/l zu 41 %

Lovastatin hemmt die Cholesterin-Biosynthese bei $10^{-7}$ mol/l zu 74% und bei $10^{-8}$ mol/l zu 48 %.

b) In Vivo-Bestimmung:

Die Hemmung der hepatischen Cholesterinbiosynthese hat Auswirkungen auf die Verminderung von Serum-Lipiden, wie im chronischen Versuch an der männlichen Ratte nachgewiesen werden kann. Gruppen männlicher Ratten des Stammes HOE: WISKf (SPF 71) mit einem Ausgangsgewicht von etwa 240 g erhalten täglich morgens die Prüfpräparate in Polyethylenglykol 400 per Schlundsonde, wobei die jeweilige Kontrolgruppe nur das Vehikel erhielt. 24 Stunden nach der letzten Applikation und nach 24 Stunden Futterentzug wurde Blut entnommen und im gewonnenen Serum die Lipoproteine aus dem Pool einer Rattengruppe mittels der präparativen Ultrazentrifugen-Technik getrennt. Hierbei wurden folgende Dichtegrenzen für die Trennung von VLDL, LDL und HDL benutzt:

VLDL 1.006

LDL 1.04

HDL 1.21

Für die Bestimmung des Cholesterins und der Triglyceride wurden vollenzymatische Methoden nach Boehringer/Mannheim benutzt, für die Bestimmung des Proteins die Methode von Lowry et al. herangezogen.

Die gemessenen Werte für die Verbindungen der Formel I im Vergleich zu Clofibrat sind im folgenden aufgeführt:

Biologische Daten

| Verbindung | Dosis | Totalcholesterin | | Protein | | Glyz | | Cholesterin |
|---|---|---|---|---|---|---|---|---|
| | mg/kg | VLDL | LDL | HDL | VLDL | LDL | VLDL | HDL/LDL |
| Formel I | 30 | -26 | -27 | +8 | -16 | -18 | -27 | 1,49 |
| Clofibrat | 100 | -54 | -32 | -28 | -8 | -9 | -10 | 1,05 |

5.

a) Charakterisierung der Verbindung der Formel I

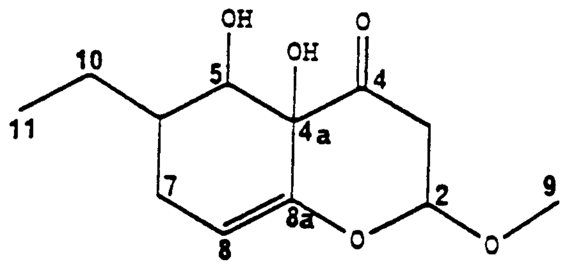

Agistatin A (Abbildung) wurde als amorpher Feststoff isoliert und ist löslich in $CH_2Cl_2$, $CHCl_3$, Ether, Essigsäureethylester, Aceton, Ethanol und Isopropanol. Agistatin A ist unlöslich in n-Pentan, n-Hexan und Cyclohexan.
Schmelzpunkt: 73° C

$$\text{Drehwert: } [\alpha]^{20}_{D} = -36{,}2 \text{ (c = 0,9; Methanol)}$$

Dünnschichtchromatographie:
Kieselgel 60, F254: Chloroform/Methanol (9:1, v:v): Rf 0,70
n-Butanol/Essigsäure/Wasser (Oberphase) (4:1:5, v:v:v): Rf 0,75
Anfärbeverhalten: Orcin: braun; Anisaldehyd-Schwefelsäure: braun.
EI-MS: m/e = 242 (8 %, $M^+$, Hochauflösung: 242.1154, $C_{12}H_{18}O_5$), 224(10 %, M-$H_2O$), 210 (10%), 182 (32 %), 168 (46 %), 140 (24 %), 139 (24 %), 111(64 %), 85 (99 %).
Molmasse: 242,27 ($C_{12}H_{18}O_5$)
IR (KBr): 3420, 2960, 2920, 1730, 1680 $cm^{-1}$
UV (MeOH): $\lambda$ = 203 nm ($\epsilon$ = 3250); 224 (1300), 261 (1000) nm
+ HCL: $\lambda$ = 203 ($\epsilon$ = 4450); 224, (1750), 271 (900) nm
+ NaOH: $\lambda$ = 211 ($\epsilon$ = 4700), 271 (1600), 303 (1450) nm
1H-NMR (200 MHz, $CDCl_3$):
$\delta$ = 0,98 (t, 3H, J = 7,0 Hz, 11-$H_3$); 1,32-1,64 (m, 2H, 10-$H_2$), 1,85 (m, 1H, 6-H); 1,94-2,26 (m, 2H, 7-$H_2$); 2,44 (d, 1H, $J_{5\text{-}OH,5}$ = 3,5 Hz, 5-OH); 2,62 (dd, 1H, $J_{3a,3b}$ = 14,5 Hz, $J_{3a,2}$ = 2,5 Hz, 3-Ha); 2,76 (s, 1H, 4a-OH); 3,32 (dd, 1H, $J_{3b,3a}$ = 14,5 Hz, $J_{3b,2}$ = 4 Hz, 3-Hb); 3,42 (s, 3H, 9-$H_3$); 4,09 (dd, 1H, $J_{5,5\text{-}OH}$ = 3,5 und J = 3,5 Hz, 5-H); 5,13 (dd, 1H, $J_{2,3b}$ = 4 Hz, $J_{2,3a}$ = 2,5 Hz, 2-H); 5,61 (dd, 1H, J = 2,5 und 5,0 Hz, 8-H).
$^{13}$C-NMR (50 MHz, $CDCl_3$):
$\delta$ = 11,6 (q, C-11); 24,3 (t, C-10); 26,1 (t, C-7); 34,6 (d, C-6); 43,8 (t, C-3); 55,6 (q, C-9); 71,5 (d` C-5), 73,6 (s, C-4a); 100,0 (d, C-2); 115,3 (d, C-8); 146,1 (s, C-8a); 205,5 (s, C-4).

| | | | |
|---|---|---|---|
| Elementaranalyse: | Berechnet:<br>Gefunden: | C 59,53<br>C 59,56 | H 7,43<br>H 7,38 |

b) Charakterisierung der Verbindung der Formel I:

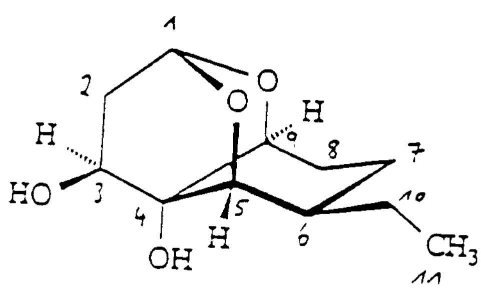

Agistatin B (Abbildung):

| | |
|---|---|
| Bruttoformel: | $C_{11}H_{18}O_4$ |
| Molmasse: | 214,26 |
| Konsistenz: | farblos, kristallin |
| Löslich in: | MeOH, EtOH |
| Unlöslich in: | n-Pentan, n-Hexan |
| Schmelzpunkt: | 161 ° C |
| IR (KBr): | 3440, 3400, 2940, 1120, 1085, 930 $cm^{-1}$ |

| Elementaranalyse: | | gefunden | berechnet |
|---|---|---|---|
| | C | 61.46 | 61.66 |
| | H | 8.39 | 8.47 |

Drehwert (MeOH): $[\alpha]^{20}_D$ = + 38,2; (c = 1,0)

Massenspektrum: m/z = 214 (22 %, $M^+$, Hochauflösung: 214.1205, $C_{11}H_{18}O_4$) 196 (4 %, M-18), 186 (4 %, M-28), 170 (6 %, M-44), 167 (14 %, M-47), 142 (66 %, M-72), 113 (66 %, M-101), 95 (99 %, M-119)

$^{13}$C-NMR (50,3 MHz, $CD_3OD$):

$\delta$ = 11,9 (q, C-11); 22,2 (t, C-10); 26,1 (t, C-7); 26,6 (t, C-8); 37,6 (d, C-6); 40,8 (t, C-2); 67,4 (s, C-4); 69,8 (d, C-3); 71,5 (d, C-5); 74,5 (d, C-9); 91,2 (d, C-1)

$^1$H-NMR (500 MHz, $CDCl_3$):

$\delta$ = 0,94 (t, 3H, $J_{11,10}$ = 7,5 Hz, 11-$H_3$); 1,30-1,80 (m, 7H, 6-H, 7-$H_2$, 8-$H_2$, 10-$H_2$); 1,97 (ddd, 1H, $J_{2a,2b}$ = 14Hz, $J_{2a,3}$ = 3,5Hz, $J_{2a,1}$ = 2Hz, 2-Ha); 2,09 (br s, 1H, OH); 2,14 (br s, 1H, OH); 2,55 (ddd, 1H, $J_{2b,2a}$ = 14Hz, $J_{2b,3}$ = 10 Hz, $J_{2b,1}$ = 2 Hz, 2-Hb); 3,81 (m, 1H, 9-H); 3,99 (dd, 1H, $J_{3,2b}$ = 10 Hz, $J_{3,2a}$ = 3 Hz, 3-H); 4,19 (br s, 1H, 5-H); 4,96 (dd, 1H, J = 2 und 2 Hz, 1-H).

c) Charakterisierung der Verbindung der Formel I

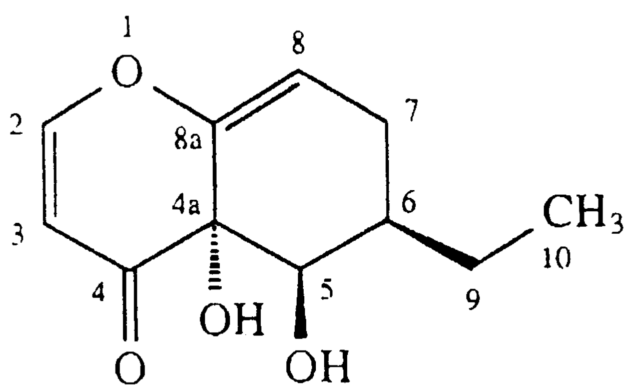

Agistatin D (Abbildung):

| | |
|---|---|
| Bruttoformel: | $C_{11}H_{14}O_4$ |
| Molmasse: | 210.23 |
| Konsistenz: | weißes Pulver |
| Löslich in: | $CH_2Cl_2$, $CHCl_3$, Ether, EtOAc, Aceton EtOH, Isopropanol |
| Unlöslich in: | n-Pentan, n-Hexan, Cyclohexan |
| Schmelzpunkt: | 93 ° C |
| UV (MeOH) nm ($\epsilon$): | 201 (3174), 227 (1132), 289 (6942) nm |
| (MeOH/HCl) ($\epsilon$): | 202 (5234), 225 (2339), 288 (7128) nm |
| (MeOH/NaOH) ($\epsilon$): | 215 (8037), 274 (4770), 305 (4937) nm |
| IR (KBr): | 3380 br, 2950, 2920, 2860, 1680, 1650, 1620, 1600, 1585, 1410, 1280, 1005 $cm^{-1}$ |
| Drehwert (MeOH): | $[\alpha]^{20}_D$ = -252,4; (c = 0,55) |
| Massenspektrum: | m/z = 210 (84 %, $M^+$) |

| Hochauflösung: | berechnet für $C_{11}H_{14}O_4$<br>gefunden | 210.0892<br>210.08937 |
|---|---|---|

192 (44 %, M-18), 163 (89 %, M-47), 139 (100 %, M-71), 137 (80 %, M-73), 126 (75 %, M-84), 71 (87 %, M-139), 55 (76 %, M-155)

$^{13}$C-NMR (50 MHz, $d_6$-Aceton):

$\delta$ = 11,9 (q, C-11); 25,1 (t, C-9); 26,7 (t, C-7); 35,8 (d, C-6); 70,4 (d, C-5); 72,1 (s, C-4a); 105,5 (d, C-3); 117,4 (d, C-8); 150,2 (s, C-8a); 159,4 (d, C-2); 192,1 (s, C-4)

$^{1}$H-NMR (200 MHz, $CDCl_3$):

$\delta$ = 0,98 (t, 3H, J = 7,0 Hz, 10-$H_3$); 1,36-1,72 (m, 2H, 9-$H_2$); 1,95 (m, 1H, 6-H); 2,04-2,38 (m, 2H, 7-$H_2$); 2,50 (s, 1H, 5-OH); 2,82 (s, 1H, 4a-OH); 4,25 (s, 1H, 5-H); 5,54 (d, 1H, $J_2$ = 6,0 Hz, 3H); 6,00 (dd, 1H, J = 2,5 und 5,0 Hz, 8-H); 7,45 (d, 1H, $J_3$ = 6,0 Hz, 2H)

d) Charakterisierung der Verbindung der Formel I

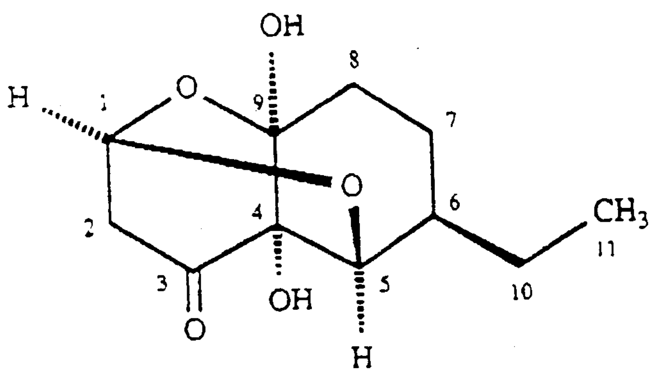

Agistatin E (Abbildung):

| | |
|---|---|
| Bruttoformel: | $C_{11}H_{16}O_5$ |
| Molmasse: | 228.24 |
| Konsistenz: | farblos, kristallin |
| Löslich in: | MeOH, EtOH, Aceton, $CHCl_3$ |
| Unlöslich in: | n-Pentan, n-Hexan, n-Heptan |
| Schmelzpunkt: | 124 °C |
| UV (MeOH/NaOH) nm ($\epsilon$): | 214 (2929), 266 (14798), 304 (3794) nm |
| IR (KBr): | 3430, 2970, 2940, 1745, 1460, 1435, 1350, 1270 $cm^{-1}$ |
| Drehwert (MeOH): | $[\alpha]^{20}_D$ = + 107,7; (C = 0,44) |
| Massenspektrum: | m/z = 228 (4 %, $M^+$) |

| Hochauflösung: | berechnet für $C_{11}H_{16}O_5$<br>gefunden | 228.09972<br>228.09972 |
|---|---|---|

210 (4 %, M-18), 181 (17 %, M-47), 164 (14 %, M-64), 140 (54 %, M-88), 139 (52 %, M-89), 111 (62 %, M-117), 83 (79 %, M-145), 55 (71 %, M-173), 43 (100 %, M-185)

$^{13}$C-NMR (50.3 MHz, $CDCl_3$):

$\delta$ = 11,6 (q, C-11); 22,2 (t, C-7); 24,2 (t, C-10); 32,4 (t, C-8); 35,4 (d, C-6); 44,6 (t. C-2); 74,8 (d. C-5); 75,5 (s, C-4); 92,3 (d, C-1); 98,2 (s, C-9); 206,4 (s, C-3)

$^{1}$H-NMR (200 MHz, $CDCl_3$):

$\delta$ = 0,92 (t, 3H, J = 7,0 Hz, 11-$H_3$); 1,40 (m, 2H, 10-$H_2$); 1,60 (m, 1H, 6-H); 1,65 (m, 2H, 7-$H_2$); 1,75/1,98 (m, 2H, 8-$H_2$), 2,70 (s 1H, 9-OH); 2,83/2.90 (dd. 2H, $J_{A,B}$ = 19,0 Hz, $J_{A,1}$ = $J_{B,1}$ = 2,0 Hz, 2-$H_A$ und 2-$H_B$), 3,74 (s, 1H, 4-OH); 3,94 (s, 1H, 5-H); 5,48 (dd. 1H, $J_{1,2-HA}$ = $J_{1,2-HB}$3 = 2,0 Hz, 1-H)

**Patentansprüche**

**1.** Die Verbindungen der allgemeinen Formel I,

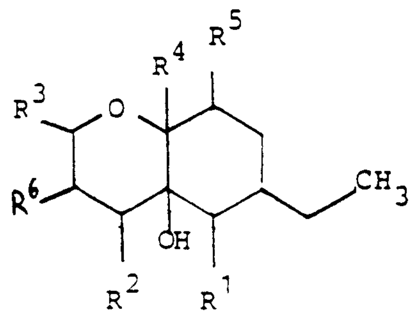

I

in der

| | |
|---|---|
| $R^1$ | Wasserstoff oder Hydroxyl ist |
| $R^2$ | Hydroxyl oder eine Oxogruppe bedeutet |
| $R^3$ | Methoxy oder Hydroxyl bedeutet oder zusammen mit $R^1$ eine Etherbrücke bildet und |
| $R^4$ | Wasserstoff oder Hydroxyl ist |
| $R^5$ | Wasserstoff bedeutet oder zusammen mit $R^4$ eine zusätzliche Bindung darstellt und |
| $R^6$ | Wasserstoff oder zusammen mit $R^3$ eine zusätzliche Bindung ist. |

**2.** Das Verfahren zur Herstellung der Verbindungen der Formel I nach Anspruch 1, dadurch gekennzeichnet, daß Fusarium sp. DSM 5983 in einem Nährmedium kultiviert wird, bis sich die Verbindungen der allgemeinen Formel I in der Kultur anhäufen.

**3.** Verfahren nach Anspruch 2, dadurch gekennzeichnet, daß das Nährmedium 0,2 bis 5 % Malzextrakt, 0,1 bis 0,5 % Hefeextrakt, 0,5 bis 2 % Glucose und 0,01 bis 0,1 % $(NH_4)_2HPO_4$ enthält, jeweils bezogen auf das Gewicht der gesamten Nährlösung.

**4.** Verfahren nach Anspruch 2 oder 3, dadurch gekennzeichnet, daß die Kultivierung bei 18 bis 30 °C erfolgt.

**5.** Verfahren nach einem oder mehreren der Ansprüche 2 bis 4, dadurch gekennzeichnet, daß die Kultivierung in dem pH-Bereich zwischen 1 und 7 erfolgt.

**6.** Verwendung der Verbindungen der allgemeinen Formel I nach Anspruch 1 als Inhibitor der Cholesterinbiosynthese.

**7.** Fusarium sp. DSM 5983 sowie deren Varianten und Mutanten, soweit sie die Verbindungen der allgemeinen Formel I nach Anspruch 1 herstellen.